# EUROPEAN PATENT APPLICATION

(11) **EP 4 295 855 A1**
(43) Date of publication of application: **27.12.2023**
(21) Application number: 22382595.1
(22) Date of filing: 23.06.2022
(51) Int. Cl.: A61K 31/739, A61K 35/74, A61P 11/00, A61P 1/00, A61P 15/06, A61P 21/00

(54) **LIPOPOLYSACCHARIDE FOR USE IN THE PREVENTION AND/OR TREATMENT OF FETAL MALFORMATIONS**

(71) Applicant: Crazy Science & Business SL, 06800 Merida (Badajoz) (ES)
(72) Inventor: LLAMAS MATÍAS, Miguel Angel, 28690 Brunete (ES); FRESNO ESCUDERO, Manuel, 28009 Madrid (ES); VALLEJO CREMADES, María Teresa, 28029 Madrid (ES)
(74) Representative: Hoffmann Eitle

(57) **Abstract**

The present invention refers to lipopolysaccharides, characterized in that they are agonist of both Toll-like Receptors TLR2 and TLR4, for use in the prevention and/or treatment of fetal malformations, preferably fetal malformations caused by malformations of muscle connective tissue such as Congenital diaphragmatic hernia (CDH).

## Description

### FIELD OF THE INVENTION

The present invention refers to the medical field. Particularly, it refers to lipopolysaccharides, characterized in that they are agonist of both Toll-like Receptors TLR2 and TLR4, for use in the prevention and/or treatment of fetal malformations, preferably fetal malformations caused by malformations of muscle connective tissue such as Congenital Diaphragmatic Hernia (CDH).

### STATE OF THE ART

CDH is a congenital disease in which the diaphragm does not develop properly and some or all parts of the abdominal organs move into the chest affecting lungs development and growth. The prevalence of CDH is approximately 2.5 cases per 10,000 births. The epidemiology of CDH is complicated by the lack of uniform data collection methods and the "hidden mortality" from unborn cases (from termination of pregnancy), stillbirths, and cases of neonatal demise before transfer to a tertiary care centre. Multi-organ morbidity is considerable among survivors and for many of them CDH is truly a chronic disease. Even more so in developing countries or in rural areas where access to pre and postnatal diagnosis and care are not readily available.

The etiology of CDH remains basically unknown since it has a multifactorial origin. Patients with CDH can be divided into two broad phenotypic categories: Isolated CDH (50-70% of cases), diagnosed when CDH is the only apparent major malformation; and Complex CDH (30-50% of cases) which is diagnosed when CDH occurs with additional abnormalities either as part of a recognized syndrome, a chromosome abnormality, or a non-syndromic constellation of major malformations. In both cases, structural abnormalities often concern cardiovascular, central nervous and musculoskeletal systems.

As previously mentioned, CDH occurs when the diaphragm is not closed properly. The most common defect is located in the posterolateral diaphragm (Bochdalek hernia - 95% of cases), while hernias involving the anterior portion of the diaphragm (Morgagni hernia) are less frequent. Bilateral herniation may occur as well, being the most rare and complicated case (less than 2%). Primarily, it was thought that the origin of the pathology was the failure of different parts of diaphragm to fuse resulted in a pleuroperitoneal canal that allowed the herniation of abdominal contents into the thorax. In the next years, an alternative theory emerged in which lung hypoplasia may be the primary causal factor in the development of CDH. A Nitrofen-induced model was used in rats and mice later, emerging a new dual hypothesis: firstly, both lungs are affected before and separates from diaphragm development (in a background of genetics and environmental factors); secondly, only an ipsilateral lung is affected after non-correct development of diaphragm resulting in herniated abdominal organs and causing lung compression (with the subsequent hypoplasia).

Genes implicated in retinol metabolism are associated with Bochdalek CDH, which is consistent with the CDH retinoid hypothesis (that says that abnormal retinoic acid signalling contributes to the development of CDH). Most of the data in support of this hypothesis has been generated from animal studies, with some supporting evidence from humans. As a new study reveals, additional support for the retinoid hypothesis by linking CDH-associated genes with the retinoid signalling pathway is provided. Several genes that were found in association with CDH have a direct role in cellular retinoic acid metabolism and signalling, further reinforcing the link between aberrant retinoic acid signalling and CDH. This includes genes encoding proteins responsible for the cellular uptake of retinol (STRA6, LRP2), cytoplasmic binding proteins of retinol and retinoic acid (RBP1, RBP2, RBP5, CRABP1, CRABP2), enzymes involved in cellular retinoic acid metabolism (LRAT, ALDH8a1), and nuclear retinoic acid receptors (RARA, RARB). This suggests that the retinoic acid signalling pathway is an important regulator of diaphragm embryogenesis and that defects in this pathway, or its downstream targets, can contribute to the development of Bochdalek CDH.

The consequences of the intrathoracic herniated organs depends on how severe the hernia's extension and duration is, which inhibits the normal growth of the lungs. This results in an abnormal differentiation of heart, lung parenchyma and vascularization and failures in the airways structures formation. Lung changes include decreased terminal branching of the bronchioles, reduced gas-exchange area and thickened alveolar walls. The pulmonary vascular bed is also affected by structural vascular remodelling with hypertrophied muscular arteries that extend more peripherally, resulting in a PPHN (Persisted Pulmonary Hypertension of the Newborn).

The importance of CDH early diagnosis cannot be overstated. Prenatal diagnosis enables antenatal counselling and informs delivery planning to optimize perinatal care. Accurate and scalable diagnostic tests are necessary to identify candidates for in utero therapeutic intervention. Ultrasound is currently the gold standard diagnostic test for CDH, but is unreliable, technically challenging and labour-intensive. Routine prenatal ultrasound identifies less than two-thirds of CDH pregnancies, especially those more severe and with associate comorbidities compared to isolated cases. The differential diagnosis for CDH includes other thoracic lesions, such as congenital pulmonary airway malformation (CPAM), bronchopulmonary sequestration (BPS), bronchogenic cyst, diaphragmatic eventration, bronchial atresia, enteric cyst, and mediastinal teratoma. Post-diagnostic evaluation aims to distinguish isolated from complex CDH, identify associated abnormalities, and establish an individualized prognosis. A combination of radiographic and genetic tests is used to stratify patients and redirect goals of care as necessary.

There is no established treatment for CDH. The current therapies are directed to prevent pulmonary hypoplasia and restore adequate lung growth for improved survival. The only fetal intervention currently offered for CDH is fetoscopy endoluminal tracheal occlusion (FETO), which involves percutaneous fetoscopy-assisted placement of a tracheal balloon at 27-29 weeks GA (Gestational Age) for severe cases of isolated CDH with O/E LHR <25% and liver herniation. The fetal observed-to expected (O/E) lung area-to-head circumference ratio (LHR) is the best-establishing measure used to as an antenatal counselling guidepost. Currently, FETO procedures are performed under maternal local anaesthetic infiltration or neuraxial anaesthesia, with or without sedation. After confirming the fetal position and placental location using ultrasonography, a fibre endoscope with a detachable balloon occlusion system is then introduced into the fetal mouth and advanced into the fetal trachea. Consequently, the balloon is inflated to occlude the trachea. Compared to historical controls, FETO increased survival in severe left-sided CDH (O/E LHR <25%) from 24% to 49% and right-sided CDH (O/E LHR <45%) from 17% to 42%. However, FETO was associated with spontaneous preterm labour and rupture of membranes in 47.1% of cases. The role of FETO is being investigated in 2 ongoing parallel Randomized Controlled Trial (RCTs) called (Tracheal Occlusion to Accelerate Lung growth [TOTAL]) in left-sided CDH fetuses with severe or moderate lung hypoplasia. Despite initial promising results, the role of FETO remains unclear and is considered experimental.

So, consequently, there is an unmet medical need of finding an effective strategy for preventing and/or treating not only CDH, but also other fetal malformations which, like CDH, are caused by malformations of muscle connective tissue. The present invention is focused on solving this problem and a new therapeutic strategy is herein proposed.

### DESCRIPTION OF THE INVENTION

As explained above, the present invention refers to lipopolysaccharides, characterized in that they are agonist of both TLR2 and TLR4 receptors, for use in the treatment of fetal malformations, preferably fetal malformations caused by malformations of muscle connective tissue such as CDH.

The inventors of the present invention have observed that both patients and animal models of CDH show an alteration in the retinoic acid pathway with a chemical or genetic origin. Modifying the immune response by polarizing the immune state of macrophages towards their M2 state could play a role in the resolution of inflammation and tissue repair, which is of interest in this study since it could positively affect the break in the diaphragm.

Moreover, the inventors of the present invention have identified that specific lipopolysaccharides which act as dual TLR2/4 ligands, at the same time, bind macrophages to promote a M2 phenotype. The M2 macrophages contribute to repair the damage associated with CDH and consequently accelerate the process of lung and/or diaphragmatic differentiation.

Importantly, such as it is shown in the Examples provided below, the treatment with a lipopolysaccharide agonist of both TLR2 and TLR4 receptors (hereinafter "LPS of the invention" or "CS1"), rescued the CDH phenotype in genetic mouse models of the disease.

So, the first embodiment of the present invention refers to an LPS, characterized in that it is agonist of both TLR2 and TLR4 receptors, for use in the prevention and/or treatment of fetal malformations.

In a preferred embodiment, the present invention refers to an LPS, characterized in that it is agonist of both TLR2 and TLR4 receptors, for use in the prevention and/or treatment of fetal malformations caused by malformations of muscle connective tissue.

In a preferred embodiment, the present invention refers to an LPS, characterized in that it is agonist of both TLR2 and TLR4 receptors, for use in the prevention and/or treatment of CDH.

In a preferred embodiment, the LPS of the invention derives from *Rhizobium spp or Ochrobactrum spp.* Please note that *Ochrobactrum* may be included in *Brucella* [Anton Hörd et al., 2020. Analysis of 1,000+ Type-Strain Genomes Substantially Improves Taxonomic Classification of Alphaproteobacteria. Front. Microbiol., 07 April 2020 | https://doi.org/10.3389/fmicb.2020.00468].

In a preferred embodiment, the LPS of the invention derives from *Rhizobium rhizogenes, Rhizobium viti or Ochrobactrum intermedium.*

In a preferred embodiment, the LPS of the invention comprises: a) O-specific polysaccharide antigen consisting of one or more repeating units, b) core oligosaccharide, and c) lipid A, wherein the lipid A is characterized in that it comprises at least 6 fatty acids and wherein at least one of the fatty acids has a chain comprising at least 24 carbon atoms, optionally with or without other substitutions.

A possible example of the structure of the LPS of the invention is represented by the **Formula I** below, wherein it is shown that at least one of the fatty acids has a chain comprising at least 24 carbon atoms in length:

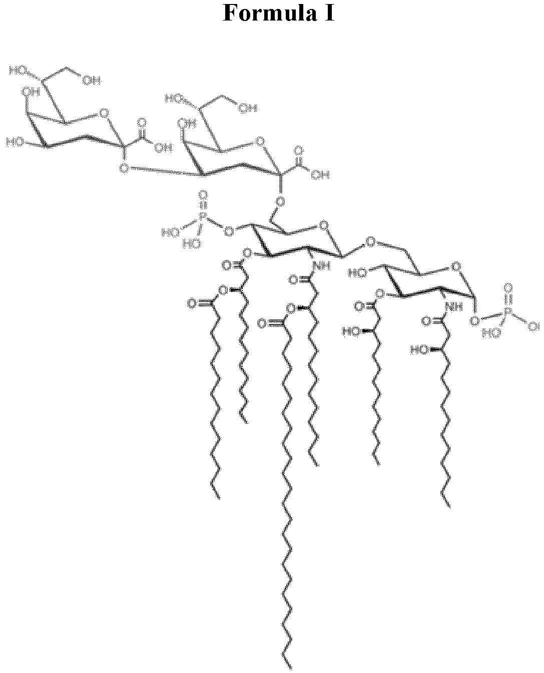

The second embodiment of the present invention refers to a pharmaceutical composition (hereinafter "pharmaceutical composition of the invention") comprising an LPS agonist of both TLR2 and TLR4 receptors and, optionally, pharmaceutically acceptable excipients or carriers, for use in the prevention and/or treatment of fetal malformations.

In a preferred embodiment, the present invention refers to the pharmaceutical composition of the invention for use in the prevention and/or treatment of fetal malformations caused by malformations of muscle connective tissue.

In a preferred embodiment, the present invention refers to the pharmaceutical composition of the invention for use in the prevention and/or treatment of CDH.

In a preferred embodiment, the pharmaceutical composition of the invention is characterized in that it comprises up to 100µg/kg of the LPS of the invention.

In a preferred embodiment, the pharmaceutical composition of the invention is characterized in that it is administered by means of a subcutaneous, subdermal, intramuscular, intraperitoneal and intravenous injection.

The last embodiment of the present invention refers to a method for the prevention and/or treatment of fetal malformations, preferably fetal malformations caused by malformations of muscle connective tissue such as CDH, which comprises administering to the patient a therapeutically effective dose or amount of the compound or pharmaceutical composition of LPS of the invention.

For the purpose of the present invention the following terms are defined:
- The term "comprising" is meant including, but not limited to, whatever follows the word "comprising". Thus, use of the term "comprising" indicates that the listed elements are required or mandatory, but that other elements are optional and may or may not be present.
- By "consisting of' is meant including, and limited to, whatever follows the phrase "consisting of'. Thus, the phrase "consisting of' indicates that the listed elements are required or mandatory, and that no other elements may be present.
- By "therapeutically effective dose or amount" is intended an amount that, when administered as described herein, brings about a positive therapeutic response in the patient. The exact amount required will vary from subject to subject, depending on (non-exhaustive list): the species, age, general condition of the subject, the severity of the condition being treated or the mode of administration. An appropriate "effective" amount in any individual case may be determined by one of ordinary skill in the art using routine experimentation, based upon the information provided herein.

### Description of the figures

**Figure 1****.** Effect of CS1 on Nitrofen-induced CDH mice.
**Figure 2****.** Left: Comparison of CD68(A), CD3(B) & CD20(C) in lungs between treatment group (CDH-) and Nitrofen control group (CDH+). P>0.05. Right: Comparison of CD68(A), CD3(B) & CD20(C) in diaphragm between treatment group (CDH-) and Nitrofen control group (CDH+). P>0.05.
**Figure 3****.** RALDH2 gene expression (measured by ARNm) in control, Nitrofen-induced CDH model and cured groups. Tissues were analyzed where CDH has more impact (lungs, diaphragm and spleen).
**Figure 4****.** RAR-a & RAR-b gene expression (measured by ARNm) in control, Nitrofen-induced CDH model and cured groups.
**Figure 5****.** RBP-1 gene expression (measured by ARNm) in control, Nitrofen-induced CDH model and cured groups.
**Figure 6****.** Arg-1 gene expression (measured by ARNm) in control, Nitrofen-induced CDH model and cured groups.
**Figure 7****.** 11-12 gene expression (measured by ARNm) in control, Nitrofen-induced CDH model and cured groups.
**Figure 8****.** CCL2 gene expression (measured by ARNm) in control, Nitrofen-induced CDH model and cured groups.
**Figure 9****.** PPAR-γ gene expression (measured by ARNm) in control, Nitrofen-induced CDH model and cured groups.
**Figure 10****.** SLIT-3 gene expression (measured by ARNm) in control, Nitrofen-induced CDH model and cured groups.
**Figure 11****.** WT-1 gene expression (measured by ARNm) in control, Nitrofen-induced CDH model and cured groups.
**Figure 12****.** Pattern of gene expression for those proteins that actively participate in inflammation and repair process. The results obtained showed variability among spleen, lung and diaphragm in control and treated and cured CDH groups.
**Figure 13****.** It illustrates that lungs belonging to treated CDH mice show an improvement on development with an increased number of respiratory bronchioles, alveolar ducts and alveoli.
**Figure 14****.** It shows the neurodifferentiation process, where mature neurons are marked by NeuN. The treatment increases the number of mature neurons at E.18 mouse embryo.
**Figure 15****.** It shows an increase of CD68 levels in rats with CDH compared to control group at E18, which is reversed by the treatment.
**Figure 16****.** It shows a significant increase of CD206 in all of groups with CDH compared to control groups, indicating a recruitment of these cells to repair tissues and solving the inflammation produced by M1.
**Figure 17****.** Normal Pleuroperitoneal folds in E10.5 mouse embryo.
**Figure 18****.** Immunofluorescence of cells from the WT1-expressing cell lineage (green). Note that WT1+ cells are more abundant at the right PHMP as compared with the left one and the lack of YFP+ cells in the central area of the ST.
**Figure 19****.** G2^{Cre}; WT1^{fl/fl} phenotype.
**Figure 20****.** Imaging showing the diaphragmatic defect and the liver invading left pleural cavity.
**Figure 21****.** Effect of CS1 in this genetic mouse model.

### Detailed description of the invention

The present invention is illustrated by means of the Examples set below without the intention of limiting its scope of protection.

### Example 1. Material and methods

### Example 1.1. CDH-induced animal models

It is well recognized that the main determinants of mortality and morbidity in CDH are related to the degree of lung underdevelopment called pulmonary hypoplasia (PH) and to the severity of pulmonary hypertension (PHTN). PH and PHTN are interlinked phenomena that are present in most patients with CDH: the disruption of signalling pathways in the fetal pulmonary epithelium, which are characteristic of PH, leads not only to abnormal lung branching morphogenesis, but also to pulmonary vascular remodelling, which is the main cause of PHTN postnatally. Over the years, research efforts have focused on modelling CDH not only to study the pathophysiology of PH and PHTN, but also to identify therapies that would promote lung growth and maturation, and correct vascular remodelling.

Animal models able to induce CDH can be divided into 3 groups: Chemical, Genetic and Surgical models.

Chemical induction models are based on the administration of teratogens or vitamin A deficiency. Nitrofen is an herbicide whose effects are directed to fetal lung development, inducing a high frequency of posterolateral and retrosternal diaphragmatic defects, as well as pericardial defects. Bisdiamine is another teratogen compound that induce CDH by inhibiting aldehyde deshydrogenase (ALDH1A2). Bisdiamine induce unilateral and bilateral CDH and the development of the fetal pulmonary mesenchyme is severely altered, resulting in abnormal blood vessel and alveolar sac formation. Vitamin A deficiency during pregnancy is another way to induce CDH in rodents as retinoic acid pathway is critical for the correct lung development. Although these and other experiments aided in defining the molecular mechanism and pathways involved in normal and abnormal fetal lung development, the delicate balance of retinoic acid must be maintained for normal embryogenesis. In fact, supplementing retinoic acid has been shown to induce apoptosis in the mesenchyme, whereas depleting retinoic acid leads to degradation of the ectoderm, and widespread teratogenesis in rodents and humans. As such, current medical practices in humans do not involve supplementing retinoic acid during pregnancy. Nonetheless, the vitamin A deficiency model has been crucial for researchers to understand the mechanism underpinning developmental anomalies.

To improve the understanding of CDH and PH pathogenesis at a molecular level, researchers have used genetic models primarily in the mouse. To date, there are 18 available mouse models of CDH with phenotypic similarity to human CDH described in the Mouse Genome Informatics Database. Among most studied are Slit3 (Slit guidance ligand 3) whose mutant mice cause have defect in the septum transversum, as also observed in human CDH 30. The gen COUP-TFII is expressed in regions critical for the formation of the diaphragm during embryonic development and its ablation in the foregut mesenchyme results in the malformation of the diaphragm. Thus, both the stomach and liver enter the thoracic cavity, leading to lung hypoplasia and neonatal death. Originally created as a model to study early urogenital development, Wilm's tumor 1 (Wt1) null mutant mice displayed diaphragmatic defects in addition to urogenital malformations. As with Nitrofen animal model, Wt1 will be treated in subsequent sections.

### Example 1.2. Nitrofen-induced model

The most widely used animal model of CDH relies on the administration of Nitrofen (2,4-dichloro-4-nitrodiphenyl ether), an herbicide that is no longer in use in agriculture. The teratogenic effects of Nitrofen are dose-dependent through either maternal feeding or maternal orogastric administration in both mice and rats. These effects can range from fetal lung development in the litter and the induction of a high frequency of posterolateral and retrosternal diaphragmatic defects, to pericardial defects. This model has shown to be valuable because it replicates pulmonary underdevelopment with a similar phenotype to that of human CDH, and with an alteration of factors and pathways that are relevant for normal human lung development. To survive, human babies with CDH require immediate intubation, mechanical intervention, and intensive care management. Likewise, pups have such severe neonatal respiratory distress at birth that they would require intubation and mechanical intervention to survive. As these intensive care manoeuvres are not feasible in the laboratory, this is considered a no surviving model.

### Example 1.3. Retinol pathway

Vitamin A plays an essential role in the embryonic development. Its deficit may drive to CDH as note in previous studies which fetuses belonging to pregnant rats with a Vitamin A-poor diet. It has also been shown a decrease of number and intensity of abnormalities after Vitamin A administration in Nitrofen murine models. It has further been reported that knockout of RBP (Retinol Binding Protein) and TTR (Trasthyretin) in animals significantly reduces retinol levels and causes embryonic abnormalities including PH 38. Moreover, retinol and RBP levels are decreased in human new-borns with CDH, whereas maternal levels were comparable between mothers of CDH patients and mothers of healthy children in a case control study. On this basis, the most robust hypothesis about CDH etiology is a disruption in the retinol signalling pathway. The problem is that we cannot afford this issue by adding Vitamin A to diet because it is a liposoluble vitamin which is toxic in higher amount and has multiple effects in fetal differentiation and development.

Although teratogen exposure causes a high incidence of CDH, coadministration with RA has a protective effect. Teratogen exposure can decrease RARE-lacZ expression relative to control, but coadministration of RA can partially reverse this effect. Raldh1 is not expressed in the Pleuroperitoneal Fold (PPF), although positive staining was identified in the adjacent lung tissue. Raldh2 is strongly expressed in the cytoplasm of cells throughout the PPF (IIB) and Raldh3 is not expressed in the PPF.

### Example 2. Results

### Example 2.1. CDH treatment with a LPS agonist of both TLR2 and TLR4 receptors (CS1)

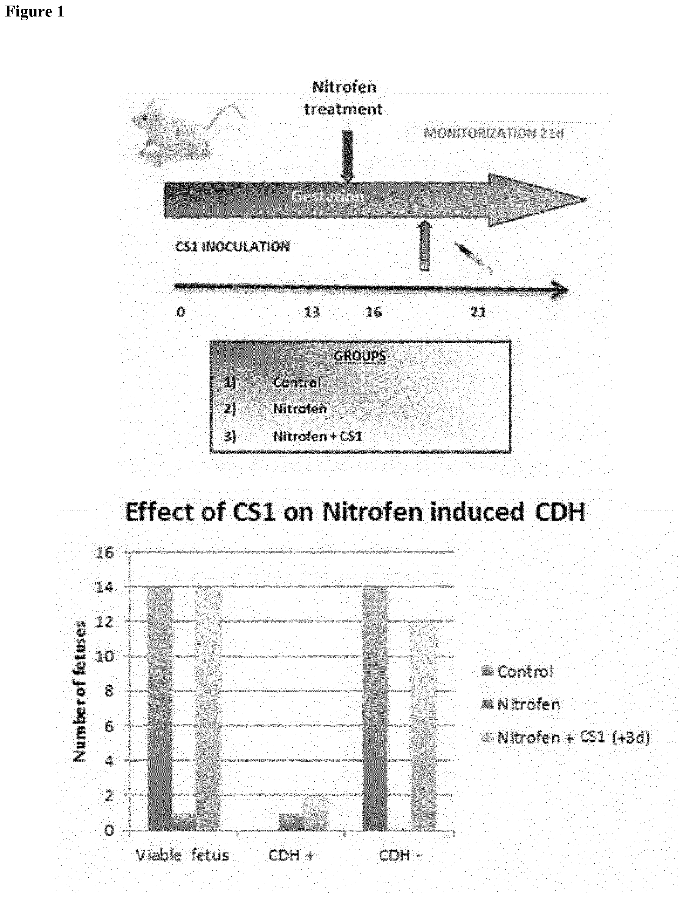

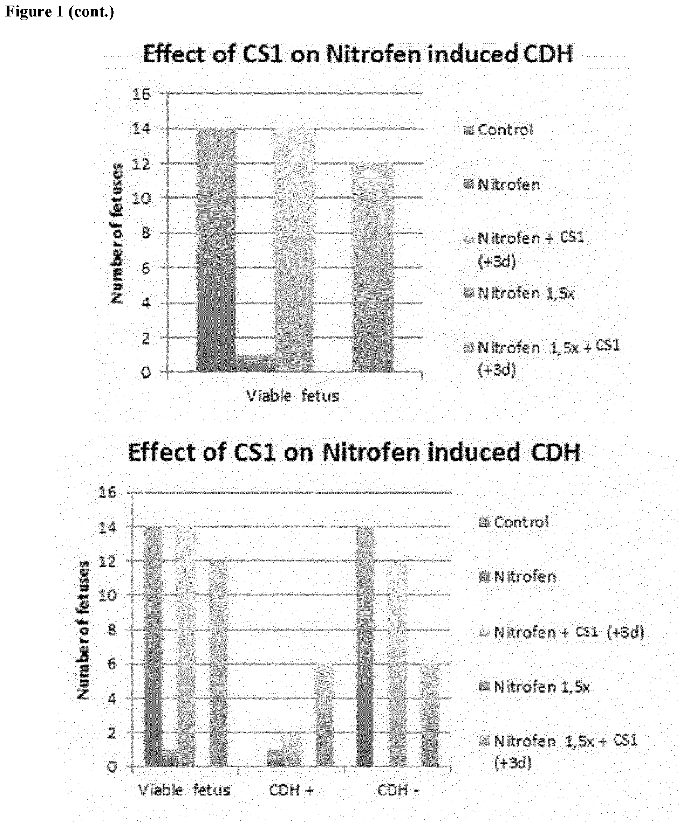

Rats treated with Nitrofen at 9.5E (days of gestation of the rat) were inoculated with CS1 immunomodulator after 3 days. While Nitrofen may induce a 60-80% of foetuses with CDH, the subsequent treatment with CS1 decreased this percentage up to 0%. In other words, CS1 is capable to reduce CDH incidence almost entirely (Figure 1). CS1 activates macrophages inducing secretion of proinflammatory and anti-inflammatory cytokines. This effect depends on TLR4 and TLR2 receptors, especially in IL-12 production.

### Example 2.2. Immune cells population in CDH

The presence of immune cells is an essential step in order to understand how these compounds act or what cells are activated through stimulation by CS1. By measuring expression markers of macrophages (CD68) and T (CD20) and B (CD3) lymphocytes with immunohistochemistry technics, we obtained the subsequent populations of these cells.

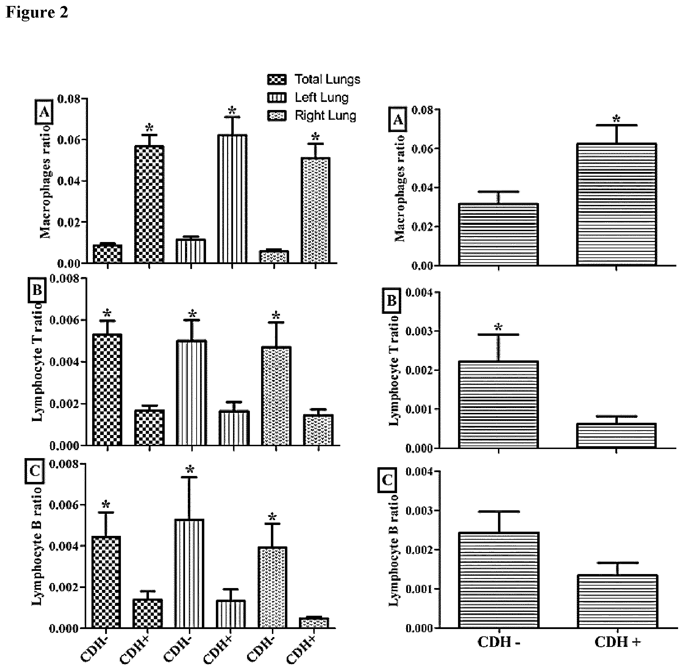

Macrophages populations are increased on both left and right lungs in rats with CDH induced by Nitrofen. The opposite happens in T and B lymphocytes populations, as there is an increase in the treated groups with CS1. Same results are showing to these cells on diaphragm tissue: low macrophages ratio and high T and B lymphocytes in rats treated with CS1 and CDH-non developed (**Figure 2**).

### Example 2.3. Gene expression measured by RT-qPCR in groups treated with Nitrofen (CDH+) and groups treated with Nitrofen + CS1 (CDH-)

Analysing different pattern of gene expression in Nitrofen and treated rats may give a better understanding of what happens in tissues where CS1 is acting. Nevertheless, it should be considered that, due to own nature of the technique, we isolated and analysed a part of a tissue that may conform different number of cells and cell subtypes in different experimental conditions. So, the volumes detected could be explained by a variation of the gene in a cell and/or an increase of cells expressing the gene concerned.

### • Retinoic acid metabolism

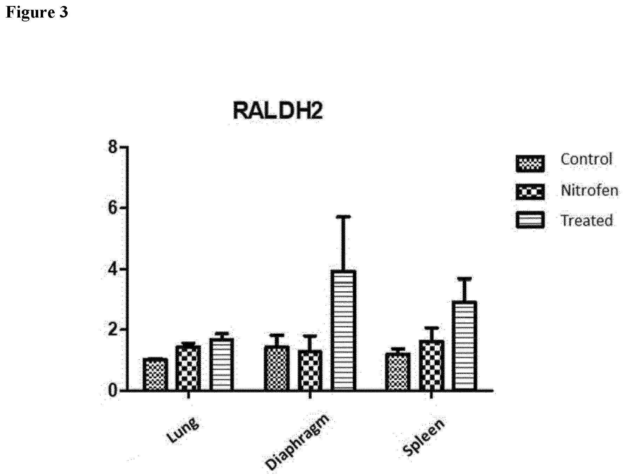

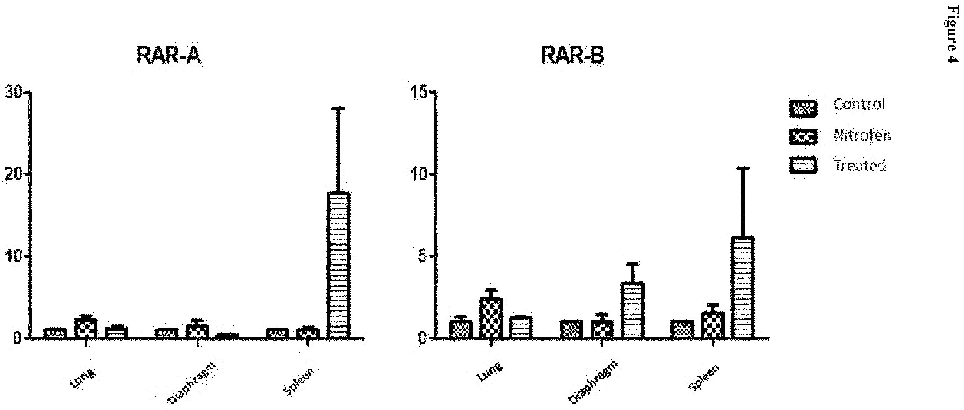

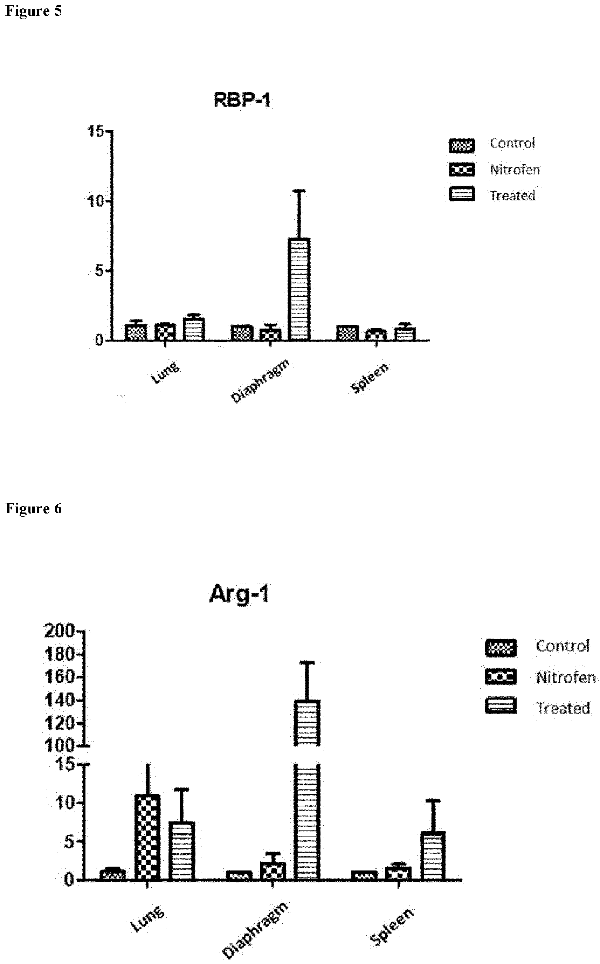

Since there is a lack of retinoic acid in infants with CDH, we analysed here different genes related with retinol metabolism:
∘ RALDH2 (Retinaldehyde Dehydrogenase Family 2): encodes a key enzyme expressed in developing lungs that catalyzes the synthesis of Retinoic Acid from retinaldehyde and it has been described that Nitrofen inhibits its action in CDH induced models 47. As we can see here, its expression is higher in cured groups (**Figure 3**).
∘ RAR-A/RAR-B: are members of the Retinoic Acid Receptors that mediate its effects in the lung cell nucleus, which also regulate gene expression by binding to short DNA sequences located near to target genes. Interestingly the expression of both genes is increased in organs without CDH, suggesting that the increased expression level of both factors could lead to the recovery of the transcription of important genes controlled by them, involved in the treatment group to reduce CDH. Also, it has been reported that double knockout mice of these genes presented significant hypoplasia and diaphragmatic defects. These genes are upregulated in treated groups, suggesting an activation of retinol pathways (**Figure 4**).
∘ RBP-1 (Retinol Binding Protein1): This gene encodes the carrier protein involved in the transport of retinol in the cytoplasm before its conversion to RA by RALDH2. Also, mutations in RBP have never been described for CDH patients. We found a small increase of RBP expression in the lungs of treated mice without CDH, being much larger in the diaphragm (**Figure 5**).

### ∘ Macrophages M2

∘ Arg-1: Arginase is an enzyme within the urea cycle in the liver and it also found in other organs such as lungs. Arg1 is predominantly expressed in myeloid cells (a marker characteristic of M2 macrophages). It has been suggested that arginase I interferes with NF-κB (critical regulator of inflammation), and consequently with inflammation affecting the immune processes reducing levels of Th2 cytokine IL-4 and IgE, being also a suppressor of T cell activation. Interestingly, we found a correlation of CDH occurrence with higher levels of this enzyme suggesting more infiltration of M2 macrophages in the damaged organs, with much higher increases in the diaphragm of CDH treated animals (**Figure 6**).

### ∘ Proinflammatory cytokines

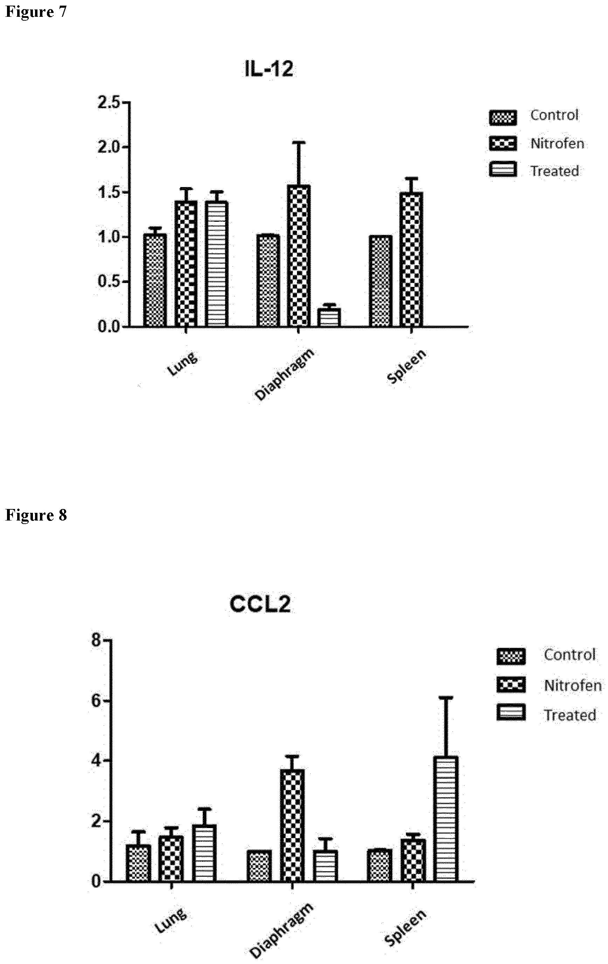

∘ Il-12: proinflammatory cytokine that plays an important role for infection control, as is capable to induce the production of CD4+ Th1 cells that mediates IFN-g, and 2) improves cytotoxicity of CD8 and NK cells. Some increases in this cytokine were observed in animal with CDH and reduced in the in the diaphragm of CDH treated animals (**Figure 7**).
∘ CCL-2 (also known as monocyte chemoattractant protein-1, MCP-1): is one of the vital chemokines that control the migration and infiltration of monocytes/macrophages. This chemokine is increased in in the diaphragm and spleen of CDH treated animals (**Figure 8**).

### ∘ Differentiation proteins/ Tissue-specific proteins

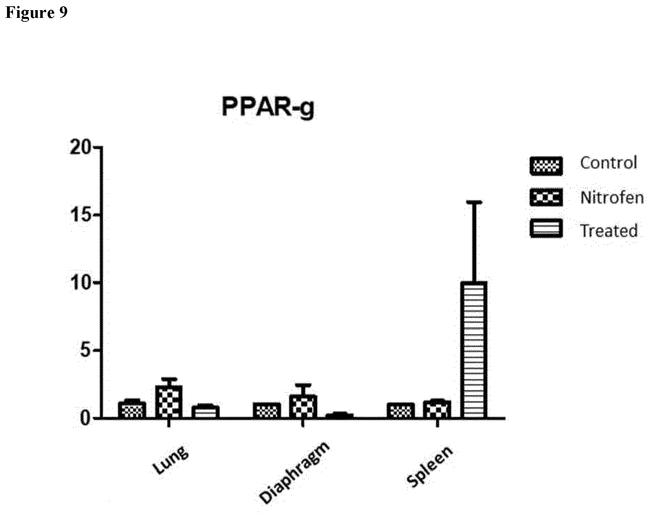

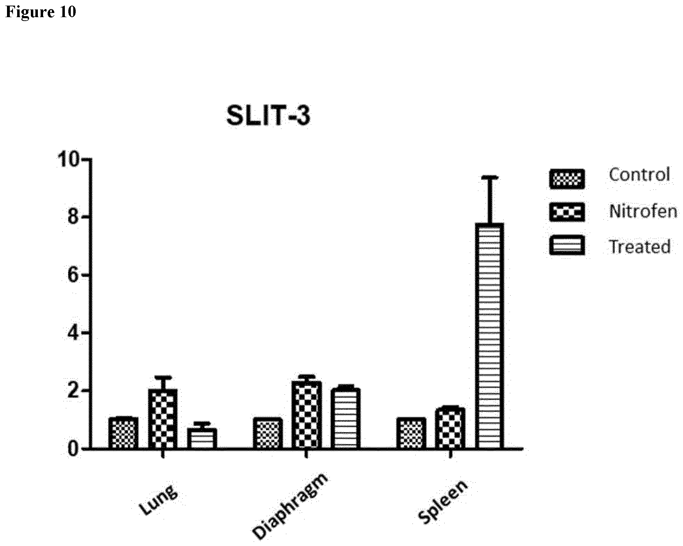

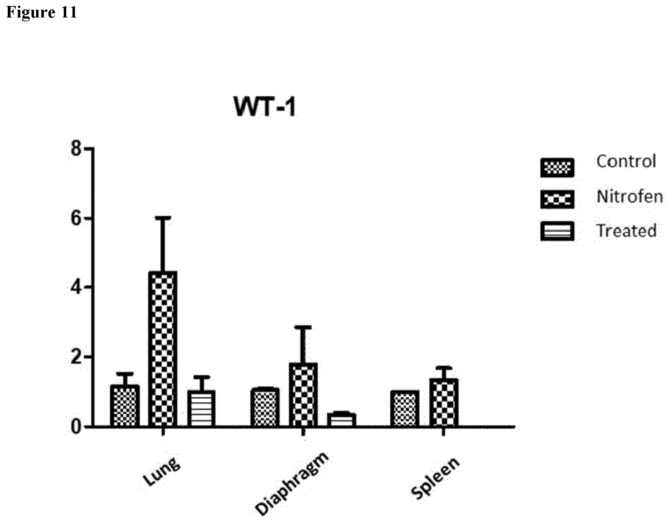

∘ PPAR γ (Peroxisome proliferator-activated receptor): it plays a key role in normal lung development. The altered pulmonary gene expression of PPARy in CDH control group confirms the detrimental effect of Nitrofen during late gestation in lung development and maturation as this gene mediates the transcription of various genes, contributing to pulmonary hypoplasia, which has statistical differences against treatment group (**Figure 9**).
∘ SLIT3 (Slit Guidance Ligand 3): is mostly expressed in diaphragm during embryonic development. Again, some increase was observed in the lungs and in the diaphragm of rats without CDH and treatment increased it in the spleen (**Figure 10**).
∘ WT-1 (Wilms' Tumor 1): transcription factor expressed during mammalian embryonic development implicated in diaphragm formation. Its conditional deletion in the lateral plate mesoderm generates up to 80% typical Bochdalek-type CDH in mice (through G2 enhancer of the Gata4 gene as a driver). WT-1 is increased in CDH and reduced by treatment (**Figure 11**).

**Table 1** summarises the expression of different genes analysed in the most important tissues affected by CDH. These results demonstrate that hernia formation alters gene expression and that CS1 treatment modulate some of these gene alterations.

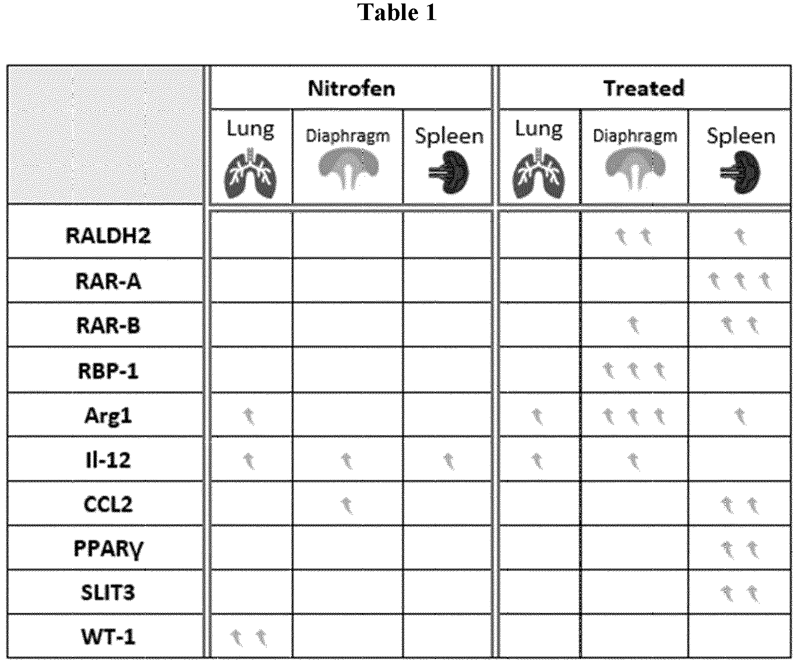

### • Macrophage's gene expression

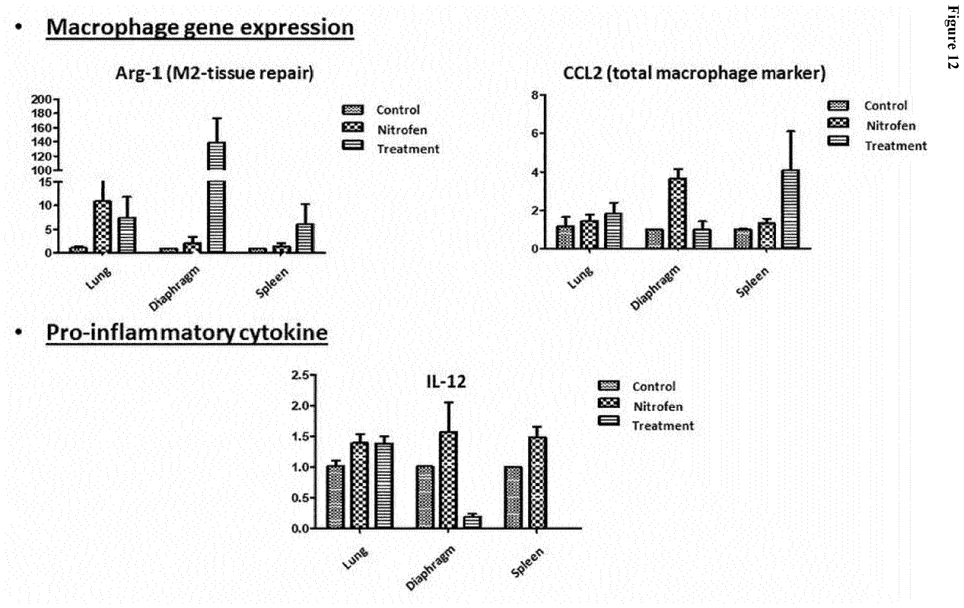

As we said before, macrophages play one of the most important roles in the occurrence of CDH. Here, we show its pattern of gene expression for those proteins that participate actively in inflammation and repair process. The results obtained showed variability among spleen, lung and diaphragm in control, treated and cured CDH groups, indicating that macrophage infiltration is modulated by damage induced by Nitrofen in the diaphragm and that M2(Arg1)/M1(IL12) ratio is favoured in the diaphragm by treatment with CS1(**Figure 12**).

### Example 2.4. CS1 restores correct lung development

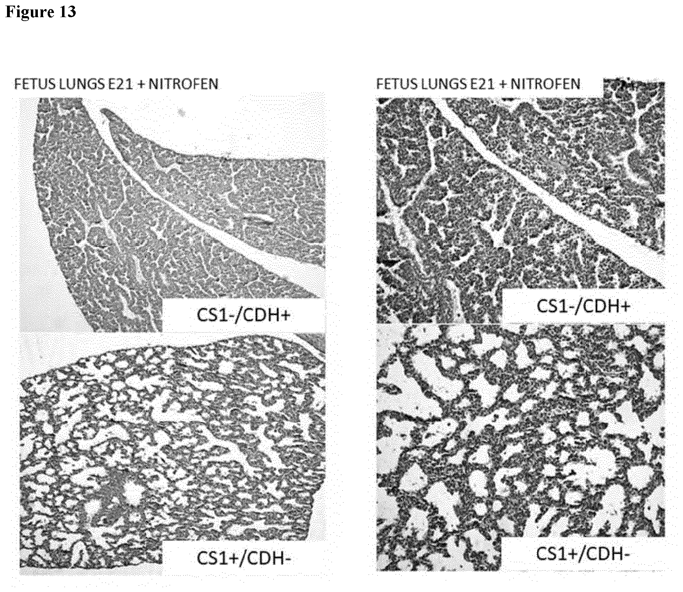

Studying anatomy of lungs from Nitrofen-induced CDH mice, treated or non-treated with CS1, we can see clear difference between groups. Lungs from treated CDH mice show an improvement on development with an increased number of respiratory bronchioles, alveolar ducts and alveoli (**Figure 13**).

### Example 2.5. Changes of inner ear

It has been reported that sensorineural hearing loss (SNHL) is prevalent among children with CDH. Up to 62% of patients with CDH may develop hearing loss, a substantially higher rate than that reported in either new-borns without complications (0.0015%-0.006%) or patients who require neonatal intensive care (1%-3%). While causes of SNHL are traditionally classified as congenital vs acquired, the mechanism in this population remains to be elucidated. Factors known to be associated with hearing loss and thought to possibly contribute to SNHL in the CDH population include ototoxic drugs, prolonged ventilation, high-frequency oscillation (HFO), neuromuscular blockade, extracorporeal membrane oxygenation, and low birth weight.

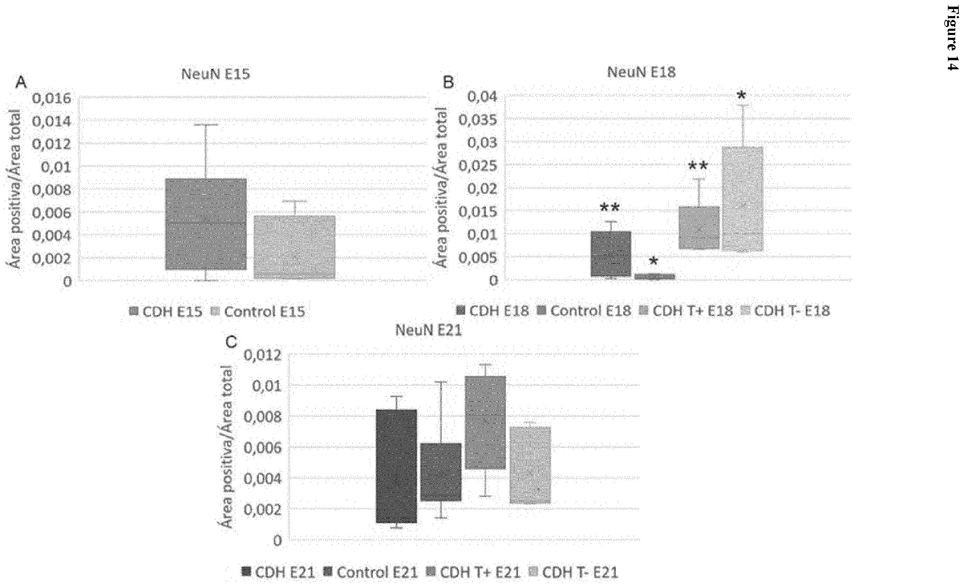

We analysed if SNHL is acquired in rats with CDH induced by Nitrofen model, and if this can be reverted by our new treatment at different developmental stages. Studying several immune cells populations from inner ear rats fetuses at E15, E18 and E21, we demonstrated that there are inner ear changes in rats with CDH and some of them can be reversed in those with CS1 treatment. The most significant variation was found in the neurodifferentiation process during embryogenesis, specifically at E-18, where the treatment increased the number of mature neurons. Additionally, we observed other changes in ear rats immune system which should be further studied to elucidate clearly the effects of CS1 in hearing loss induced by CDH (**Figure 14**).

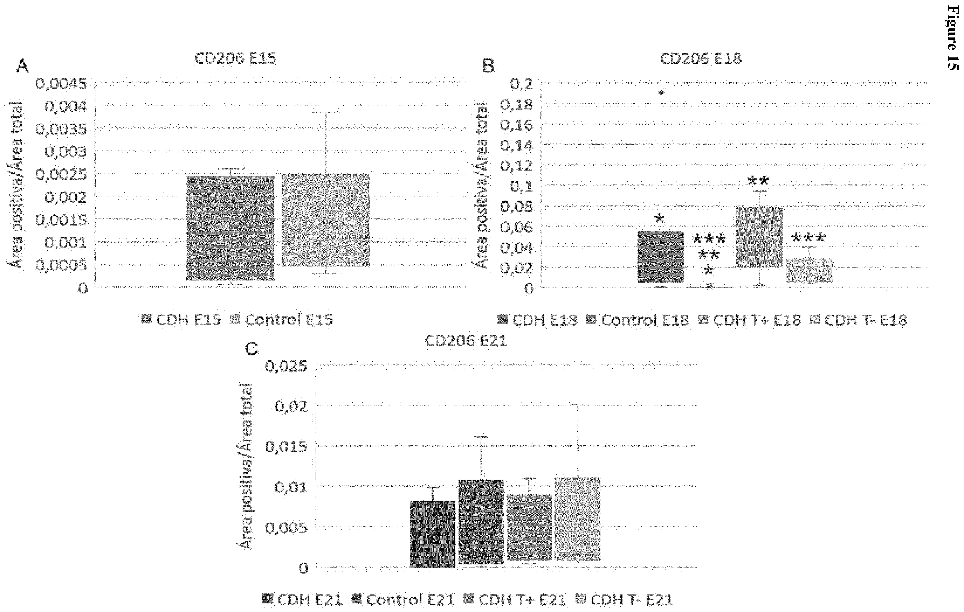

The next step was to check what happen with M2 macrophages population. We used the M2 macrophage specific marker CD206. We show above a significant increase in all of groups with CDH compared to control groups, indicating a recruitment of these cells to repair tissues and solving the inflammation produced by M1 (**Figure 15**)

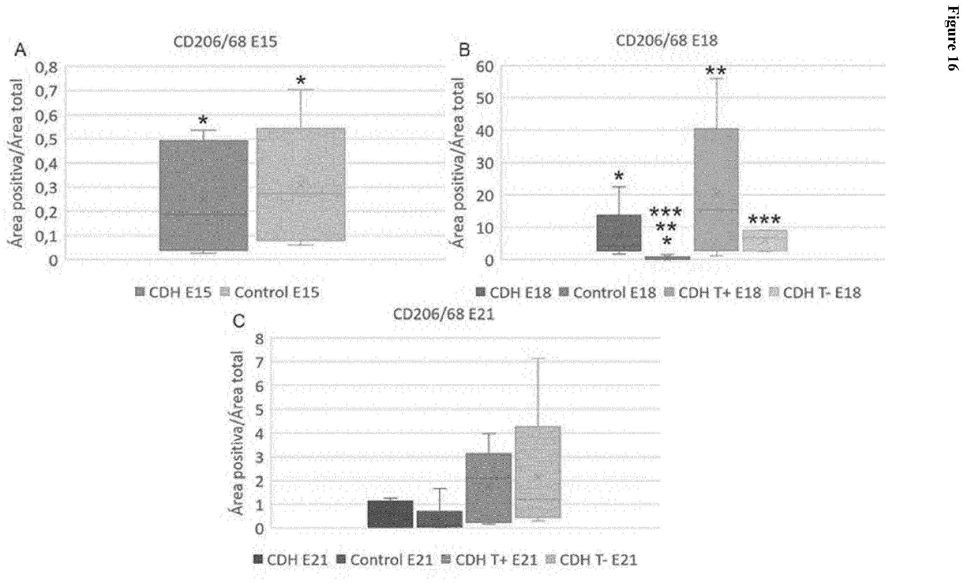

Finally, we studied macrophage activity measuring the relationship between M2 and M1 macrophages. This activity is clearly proinflammatory at E15 as indicated in the graph A of **Figure 16****.** Drastic changes are produced at E18 in all of groups, where M2 play an essential role to repair damage tissue and solve the inflammation.

### Example 2.6. Genetic mouse model (G2^{cre}; WT1^{fl/fl}) to induce CDH

To further study the efficiency of CS1 we used a novel transgenic mouse to prove that these compounds act on the disease, regardless its etiology.

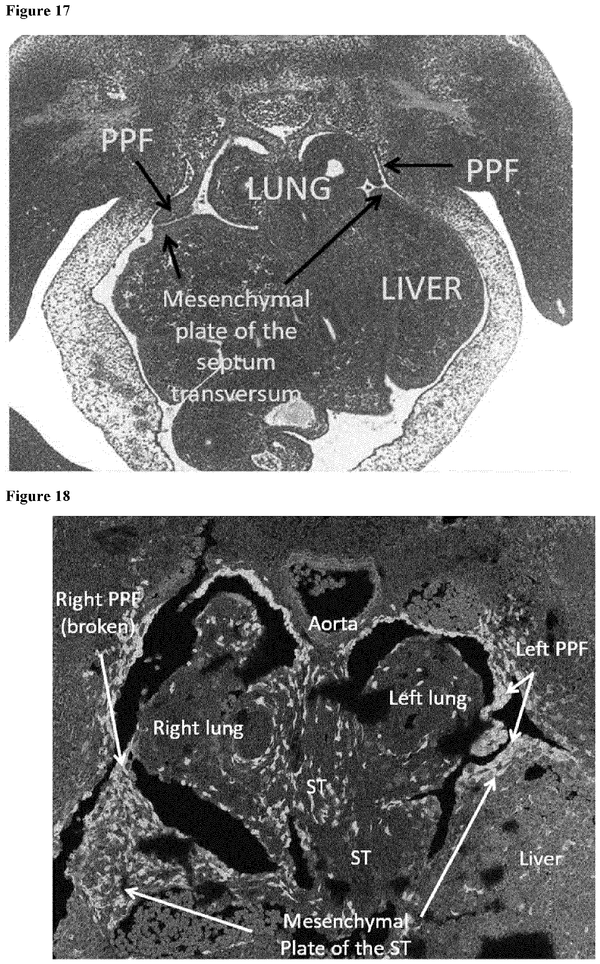

To understand the proposed CDH-induced model, we need to take into account the anatomical changes produced at the chest level, mainly in the diaphragm formation. In the Bochdalek-CDH type (also called posterolateral hernia; 80-90% of all cases of CDH), the classical hypothesis postulates a failure in the fusion of pleuroperitoneal folds (PPFs) with the septum transversum (ST). Some authors describe that this fusion rather occurs with the posthepatic mesenchymal plate (PHMP), an accumulation of mesenchymal cells derived from the ST and located in the posterodorsal margin of the liver lobes (**Figure 17**).

To study the role of WT1 in CDH, we performed loss-of-function experiments by conditionally inactivating the Wt1 gene in the ST/PHMP/PPFs mesenchyme. The use of WT1 conditional knockout overcomes the early embryonic death caused by systemic deficiency of WT1. We used a driver based on the G2 enhancer of the Gata4 gene. This enhancer drives expression of Gata4 in the lateral plate mesoderm from the stage E7.5, and by the stage E9.5 is active in the septum transversum and proepicardium, ceasing its activity by E12.5. The activity of this enhancer is completely absent in the intermediate mesoderm. Our findings indicate that WT1 is involved in the generation of the mesenchyme of the ST/PHMP/PPFs continuum through epithelial-mesenchymal transition and they provide a novel perspective on the genesis of the Bochdalek hernia and the evolutionary origin of the diaphragm (80% of *G2-Gata4^{Cre}; Wt1*^{*fl*/*fl*} embryos developed typical Bochdalek-type CDH).

WT1 was expressed both mesenchymal margins of the liver (PHMP) and PPF in embryos at E10.5, showed by *Wtl^{Cre}; R26^{EYFP},* a mouse model where WT1 positive cells are traced through YFP label (**Figure 18**).

However, differences in the number of mesenchymal cells in the PHMP or ST had not been reported, although some authors pointed to a larger surface contact between PPFs and PHMP at the right side. I has been shown that the right part of the PHMP shows more mesenchymal cells than the left part by the time in which the pleuroperitoneal septa develop, and we believe that this fact is relevant for the left predominance (>85%) of the Bochdalek CDH.

### Example 2.6.1. Effect of CS1 in the genetic model of CDH G2^{Cre}; WT1^{fl/fl} phenotype

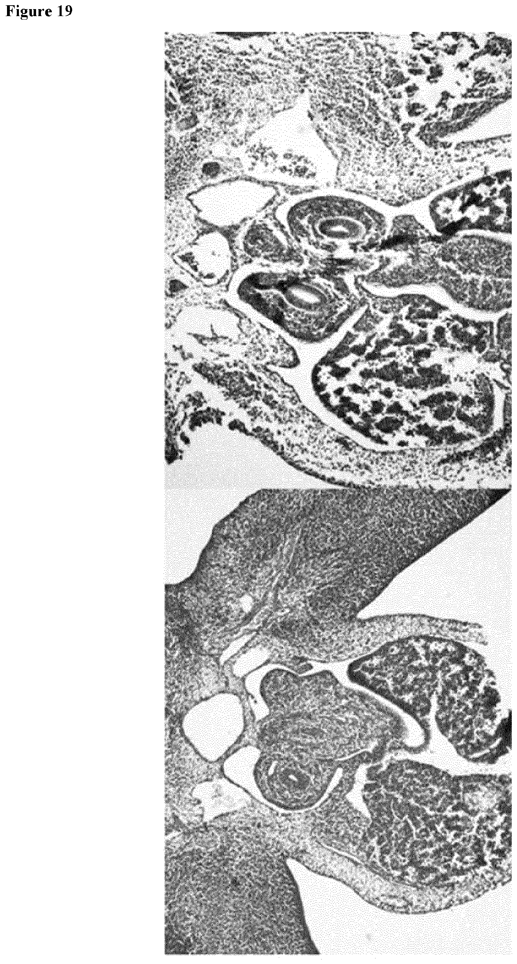

This genetic model is characterized in that (**Figure 19**):
- Strong reduction of the ST mesenchyme, in embryos at E10.5.
- Liver invades left pleural cavity and this lung becomes to hypoplasic (E14.5).
- Diaphragmatic defects by lacking the continuity (E14.5).

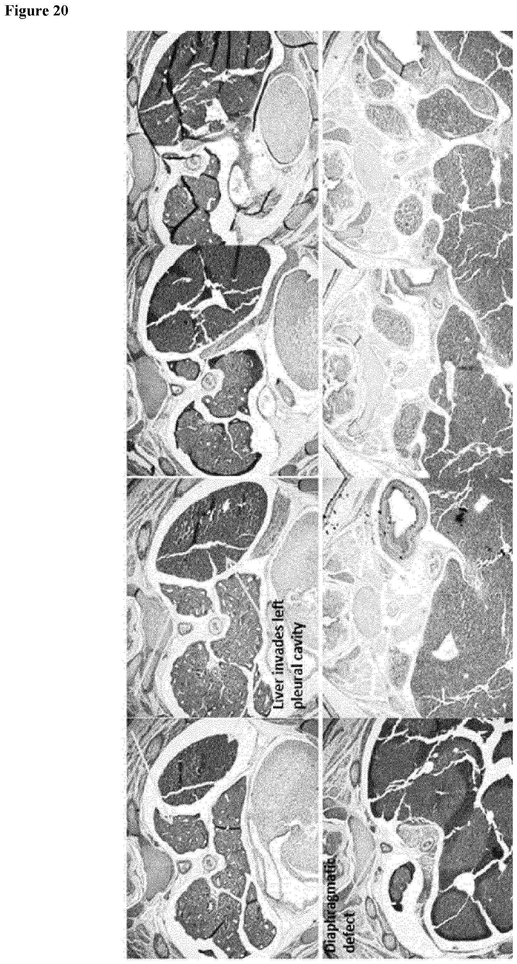

The researchers hypothesized that ST mesenchyme seems to migrate into the PPF during the fusion. In mutant embryos, there is not enough mesenchyme to achieve this migration, fusion fails, and pleural cavities (usually the left one) remain open, resulting in a Bochdalek-type CDH (**Figure 20**).

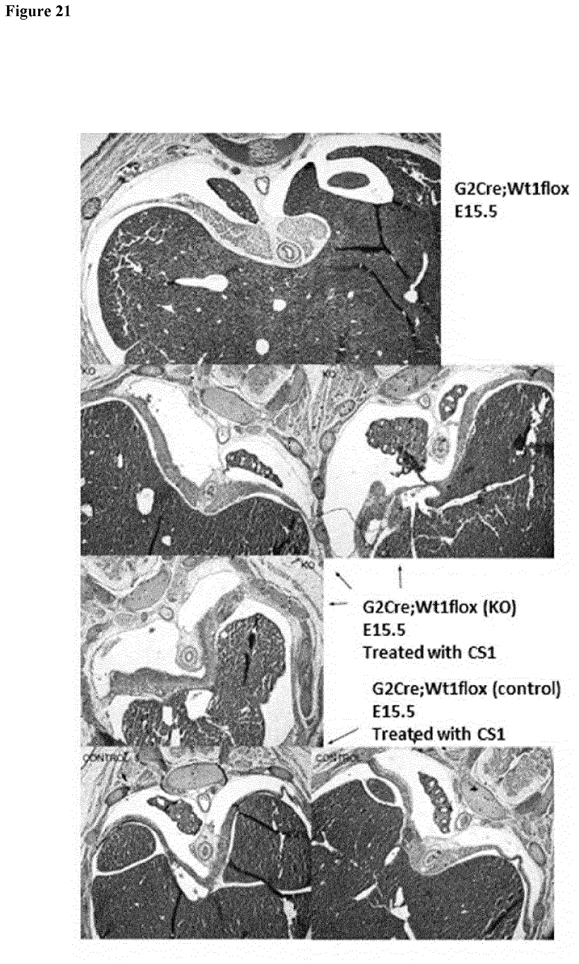

CS1 treatment rescued the CDH phenotype in this purely genetic model. Only 1 out 11 mutant embryos treated with CS1 showed CDH (9,1%), but normal prevalence of the phenotype in non-treated mutant embryos is 70%. In some cases, the diaphragm appeared thicker, but is completely closed anyhow. It is important to say that control embryos treated with CS1 were normal (**Figure 21**).

## Claims

1. Lipopolysaccharide, **characterized in that** it is agonist of both TLR2 and TLR4 receptors, for use in the treatment of fetal malformations.

2. Lipopolysaccharide for use, according to claim 1, in the treatment of fetal malformations caused by malformations of muscle connective tissue.

3. Lipopolysaccharide for use, according to any of the claims 1 or 2, in the treatment of Congenital Diaphragmatic Hernia.

4. Lipopolysaccharide for use, according to any of the previous claims, wherein the lipopolysaccharide derives from *Rhizobium spp or Ochrobactrum spp.*

5. Lipopolysaccharide for use, according to any of the previous claims, wherein the lipopolysaccharide derives from *Rhizobium rhizogenes, Rhizobium viti or Ochrobactrum intermedium.*

6. Lipopolysaccharide for use, according to any of the previous claims, which comprises: a) O-specific polysaccharide antigen consisting of one or more repeating units, b) core oligosaccharide, and c) lipid A, wherein the lipid A is **characterized in that** it comprises at least 6 fatty acids and wherein at least one of the fatty acids has a chain comprising at least 24 carbon atoms, optionally with or without other substitutions.

7. Pharmaceutical composition comprising a lipopolysaccharide agonist of both TLR2 and TLR4 receptors and, optionally, pharmaceutically acceptable excipients or carriers, for use in the treatment of fetal malformations.

8. Pharmaceutical composition for use, according to claim 7, in the treatment of fetal malformations caused by malformations of muscle connective tissue.

9. Pharmaceutical composition for use, according to any of the claims7 or 8, in the treatment of CDH.

10. Pharmaceutical composition for use, according to any of the claims 7 to 9, wherein the lipopolysaccharide derives *Rhizobium spp* or *Ochrobactrum spp.*

11. Pharmaceutical composition for use, according to any of the claims 7 to 10, wherein the lipopolysaccharide derives from *Rhizobium rhizogenes, Rhizobium viti* or *Ochrobactrum intermedium.*

12. Pharmaceutical composition for use, according to any of the claims 7 to 11, **characterized in that** it comprises up to 100µg/kg of the lipopolysaccharide.

13. Pharmaceutical composition for use, according to any of the claims 7 to 12, **characterized in that** it is administered by means of a subcutaneous, subdermal, intramuscular, intraperitoneal and intravenous injection.
